# EUROPÄISCHE PATENTSCHRIFT

(11) **EP 1 661 592 B1**
(45) Veröffentlichungstag und Bekanntmachung des Hinweises auf die Patenterteilung: **28.05.2008**
(21) Anmeldenummer: 04027855.8
(22) Anmeldetag: 24.11.2004
(51) Int. Cl.: A61M 1/36

(54) **Vorrichtung zur Bereitstellung eines extrakorporalen Blutkreislaufs**
Device for providing an extracorporeal blood circuit
Dispositif pour fournir un circuit sanguin extracorporel

(43) Veröffentlichungstag der Anmeldung: 31.05.2006
(60) Teilanmeldung: 07010455.9
(73) Patentinhaber: LIFEBRIDGE Medizintechnik AG, 84539 Ampfing (DE)
(72) Erfinder: Brieske, Gerhard, 84539 Ampfing (DE)
(74) Vertreter: Manitz, Finsterwald & Partner GbR

(56) Entgegenhaltungen:
- WO-A-20/04098678
- DE-C1- 19 905 937
- US-A- 5 232 437
- US-A- 6 071 258

## Beschreibung

Die vorliegende Erfindung betrifft eine Vorrichtung zur Bereitstellung eines extrakorporalen Blutkreislaufs, insbesondere Herz-Lungen-Maschinen, umfassend zumindest ein Basismodul mit einer Steuereinrichtung und ein mit dem Basismodul lösbar verbundenes Patientenmodul, das Blut führende Komponenten des extrakorporalen Blutkreislaufs aufweist.

Derartige Herz-Lungen-Maschinen sind beispielsweise als tragbare Geräte für den Notfalleinsatz bekannt. Problematisch ist hierbei, dass bei solchen Geräten vor der Inbetriebnahme sichergestellt sein muss, dass sich in den Blut führenden Komponenten des extrakorporalen Blutkreislaufs keine Luft mehr befindet, da dies einen Patienten ernsthaft gefährden könnte. Zur Beseitigung der in den Blut führenden Komponenten vorhandenen Lufteinschlüsse wird deshalb vor der Inbetriebnahme eine so genannte Priming-Flüssigkeit eingefüllt und der extrakorporale Blutkreislauf wird entlüftet. Dies ist jedoch bei den bekannten Vorrichtungen zeitaufwendig.

Eine tragbare Herz-Lungen-Maschine gemäß dem Oberbegriff des Anspruchs 1 ist aus WO 2004/098678 bekannt.

Es ist die Aufgabe der vorliegenden Erfindung, eine Vorrichtung der eingangs genannten Art zu schaffen, mit der das Entlüften eines mit Priming-Flüssigkeit befüllten Patientenmoduls in kurzer Zeit erfolgen kann.

Die Lösung dieser Aufgabe erfolgt durch die Merkmale des Anspruchs 1 und insbesondere dadurch, dass an dem Basismodul und/oder an dem Patientenmodul Schwenkmittel vorgesehen sind, um das Patientenmodul relativ zu dem Basismodul um eine horizontale Achse von einer Befüllposition in eine Betriebsposition zu verschwenken.

Durch die erfindungsgemäß vorgesehenen Schwenkmittel lässt sich das Patientenmodul relativ zu dem Basismodul geführt verschwenken, wodurch sich auch die Lage und Orientierung einzelner Komponenten des extrakorporalen Blutkreislaufs verändert, so dass Lufteinschlüsse, die in der Befüllposition konstruktionsbedingt nicht entweichen können, bei bzw. nach Übergang in die Betriebsposition über Entlüftungsleitungen aus dem System entfernt werden können.

Aufgrund der erfindungsgemäßen Schwenkmittel kann das Befüllen und Entlüften eines Patientenmoduls in einem Zeitraum in der Größenordnung von etwa 2 Minuten erfolgen, wohingegen vergleichbare Vorrichtungen nach dem Stand der Technik für diesen Vorgang etwa 20 Minuten benötigen, was in Notfallsituationen entscheidend sein kann.

Vorteilhafte Ausführungsformen der Erfindung sind in der Beschreibung, der Zeichnung sowie den Unteransprüchen beschrieben.

Nach einer ersten vorteilhaften Ausführungsform liegen zwischen der Befüllposition und der Betriebsposition etwa 90°, was den Vorteil hat, dass etwaige Lufteinschlüsse sicher aus den Blut führenden Komponenten entweichen können.

Nach einer weiteren vorteilhaften Ausführungsform ist in dem Patientenmodul ein Blutreservoir vorgesehen, das sowohl in der Befüllposition wie auch in der Betriebsposition mit einer Neigung von etwa 45° zur Horizontalen angeordnet ist. Dies hat zur Folge, dass das Blutreservoir nach einer Drehung des Patientenmoduls um 90° wieder die gleiche Orientierung relativ zur Horizontalen besitzt, so dass sich vor und nach dem Schwenken innerhalb des Reservoirs gleiche Strömungsverhältnisse ergeben.

Nach einer weiteren vorteilhaften Ausführungsform kann in dem Patientenmodul ein Zentrifugalpumpenkopf mit zentralem Einlass und tangentialem Auslass so angeordnet sein, dass der Einlass in der Befüllposition vertikal nach oben und in der Betriebsposition horizontal orientiert ist. Auf diese Weise kann der Pumpenkopf von oben mit Priming-Flüssigkeit befüllt werden, ohne dass hierbei Lufteinschlüsse im Pumpenkopf verbleiben. Hierbei kann es ebenfalls vorteilhaft sein, den Zentrifugalpumpenkopf mit einem tangentialen Auslass zu versehen, der in der Betriebsposition an der untersten Stelle des Zentrifugalpumpenkopfes angeordnet ist. Auf diese Weise ist sichergestellt, dass im Betrieb durch die Zentrifugalpumpe keine Luft innerhalb des intrakorporalen Blutkreislaufs gepumpt wird.

Nach einer weiteren vorteilhaften Ausführungsform kann in dem Patientenmodul ein arterieller Filter mit einem Entlüfungsauslass so angeordnet sein, dass der Entlüftungsauslass in der Befüllposition horizontal und in der Betriebsposition vertikal nach oben orientiert ist. Hierdurch ergibt sich wiederum die Möglichkeit, dass Luft innerhalb des arteriellen Filters, die nach dem Befüllen mit Priming-Flüssigkeit noch im Filter vorhanden ist, nach einem Verschwenken in die Betriebsposition über den Entlüftungsauslass nach oben entweichen kann.

Die erfindungsgemäß vorgesehenen Schwenkmittel können in verschiedensten Ausführungen vorgesehen sein und insbesondere eine verschwenkbar an dem Basismodul gelagerte Aufnahme für das Patientenmodul umfassen. In diesem Fall muss das Patientenmodul lediglich mit der Aufnahme gekoppelt werden, um eine geführte Schwenkbewegung zu ermöglichen. Hierbei ist es besonders vorteilhaft, wenn die Schwenkmittel eine Führung umfassen, die an der Aufnahme und an dem Patientenmodul vorgesehen ist. In diesem Fall kann auch das Patientenmodul dazu herangezogen werden, die geführte Schwenkbewegung sicherzustellen.

Alternativ ist es möglich, das Patientenmodul mit einem weiteren Modul, beispielsweise einem Steuermodul, zu verbinden und die Einheit aus Patientenmodul und Steuermodul an der Aufnahme zu befestigen. In diesem Fall kann die Führung an der Aufnahme und an dem Steuermodul vorgesehen sein.

Auch ist es beispielsweise möglich, an dem Basismodul ein Schwenklager vorzusehen, in welches das oder die anderen Module eingesetzt werden.

Bevorzugt befindet sich das Patientenmodul nach Aufsetzen auf das Basismodul in der Betriebsposition, da in diesem Fall auch ein rasches Abnehmen des Patientenmoduls von dem Basismodul sichergestellt ist, ohne dass vorher ein Verschwenken erfolgen muss.

Nach einer weiteren vorteilhaften Ausführungsform weist das Basismodul einen Gerätefuß auf, der mit einem Klappbügel versehen ist, um die Vorrichtung an dem Rahmen eines Patientenbettes einzuhängen.

Nach einem weiteren Aspekt der Erfindung betrifft diese ein Verfahren zur Inbetriebnahme einer Vorrichtung der vorstehend genannten Art, wobei das Patientenmodul zunächst in die Befüllposition gebracht wird, in der Befüllposition die Blut führenden Komponenten mit einer Priming-Flüssigkeit befüllt werden, wobei anschließend das Patientenmodul relativ zu dem Basismodul, insbesondere um 90°, in die Betriebsposition verschwenkt wird. Mit einem solchen Verfahren ergeben sich die vorstehend geschilderten Vorteile.

Nach einer vorteilhaften Verfahrensvariante kann vor dem Verschwenken ein in dem Patientenmodul vorgesehener Pumpenkopf angetrieben werden, um die bereits eingefüllte Priming-Flüssigkeit zu pumpen und dadurch die Blut führenden Komponenten weiter zu entlüften.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: eine perspektivische Ansicht einer tragbaren Herz-Lungen-Maschine;
- Fig. 2: eine perspektivische Ansicht des Steuermoduls der Herz-Lungen-Maschine von Fig. 1, das mit einer Aufnahme des Basismoduls verbunden ist;
- Fig. 3: eine perspektivische Ansicht einiger Blut führender Komponenten des Patientenmoduls in der Befüllposition;
- Fig. 4: die Darstellung von Fig. 3 in der Betriebsposition, jedoch in einer Ansicht von hinten; und
- Fig. 5: ein Diagramm, das die einzelnen Komponenten der Herz-Lungen-Maschine darstellt.

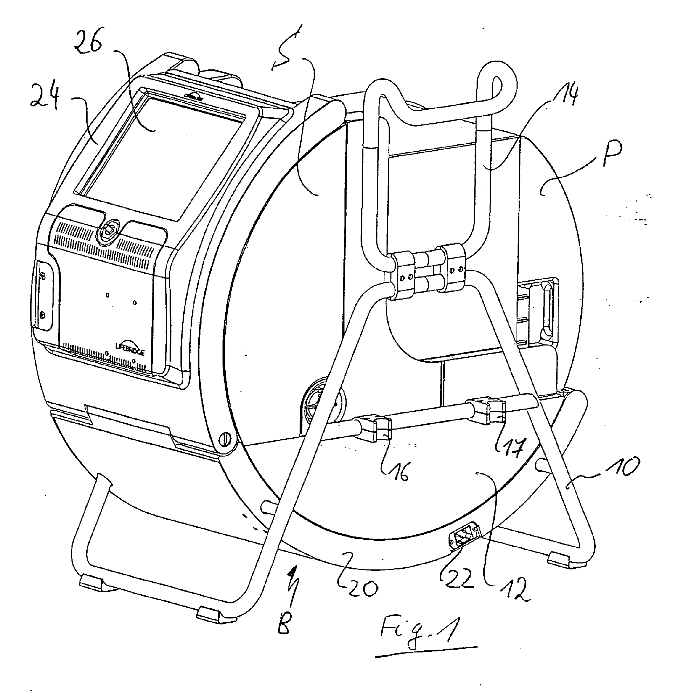

Die in den Figuren 1 bis 4 dargestellte Herz-Lungen-Maschine ist aus drei Modulen zusammengesetzt, nämlich einem mit einem Gerätefuß 10 versehenen Basismodul B, einem Steuermodul S und einem Patientenmodul P, das Blut führende Komponenten eines extrakorporalen Blutkreislaufs aufweist.

Bei der dargestellten Ausführungsform ist das Patientenmodul P über nicht dargestellte Rastelemente mit dem Steuermodul S zu einer Einheit gekoppelt und diese Einheit, bestehend aus Steuermodul S und Patientenmodul P ist auf einer Aufnahme 12 des Basismoduls B lösbar verrastet.

Wie Fig. 1 zeigt, weist der aus Rohrmaterial gefertigte Gerätefuß 10 an seiner Oberseite einen Klappbügel 14 auf, der an seiner Oberseite zu einem Haken gebogen ist, um ein Einhängen an einem Rahmen eines Patientenbettes zu ermöglichen. Von der in der Fig. 1 dargestellten Position kann der Klappbügel 14 um 180° nach unten verschwenkt werden und in zwei Halteklipse 16, 17 eingesteckt werden, damit der Klappbügel 14 beim Aufsetzen des Steuermoduls S und des Patientenmoduls P nicht stört.

Der Gerätefuß 10 ist mit einem Trägerelement 20 des Basismoduls B fest verbunden, das eine Steckbuchse 22 für ein Netzkabel aufweist. Ferner ist die Aufnahme 12 in dem Trägerelement 20 verschwenkbar gelagert, was nachfolgend noch näher erläutert wird.

An der in der Fig. 1 linken Seite des Trägerelementes 20 ist ein Bedienteil 24 abklappbar befestigt, das einen Touchscreen 26 aufweist, der ein Ein- und Ausgabemittel für eine in dem Basismodul vorgesehene Steuereinrichtung (Computer) darstellt. Wie die Figur ferner zeigt, bildet das Trägerelement 20 und das nicht abgeklappte Bedienteil 24 einen kreisringförmigen Mantel für die Einheit aus Aufnahme 12, Steuermodul S und Patientenmodul P. Zum Aufsetzen bzw. Abnehmen der Einheit aus Steuermodul S und Patientenmodul P muss das Bedienteil 24 aus der in Fig. 1 dargestellten Position nach links aufgeklappt werden.

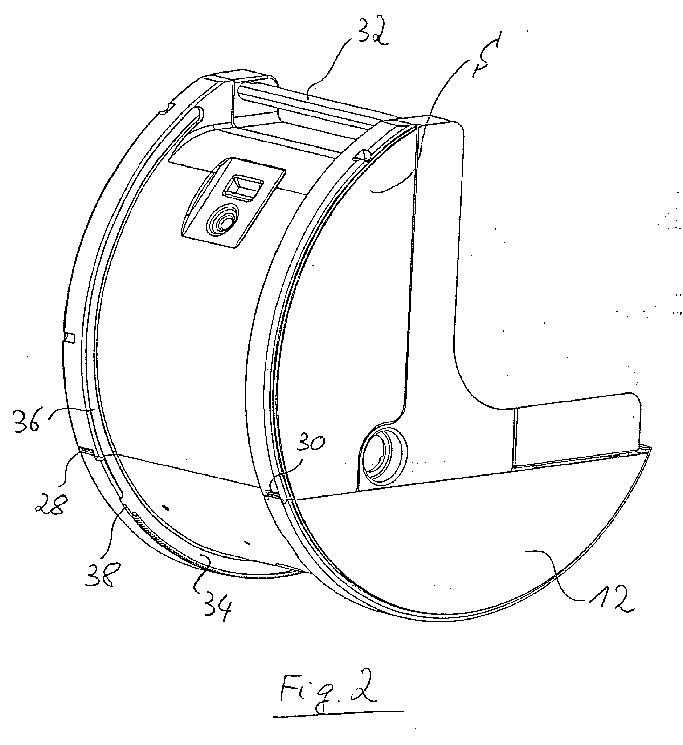

Fig. 2 zeigt die Aufnahme 12 des Basismoduls B von Fig. 1, auf die das Steuermodul S mittels Rastverbindungen 28, 30 lösbar verbunden ist. Zur vereinfachten Darstellung ist das Patientenmodul P in Fig. 2 nicht gezeigt. Es sei jedoch darauf hingewiesen, dass im Betrieb stets eine Einheit aus Steuermodul S und Patientenmodul P auf die Aufnahme 12 gesteckt bzw. von dieser entfernt wird.

Wie Fig. 2 ferner zeigt, ergänzt das Steuermodul S die scheibensegmentförmige Geometrie der Aufnahme 12 und an der Oberseite des Steuermoduls S befindet sich ein Handgriff 32, mit dem einerseits die Einheit aus Steuermodul S und Patientenmodul P andererseits aber auch die gesamte Herz-Lungen-Maschine gehandhabt werden kann, wenn die drei Module wie in Fig. 1 dargestellt aneinander befestigt sind.

Um das in Fig. 2 lediglich nicht dargestellte Patientenmodul P relativ zu dem Basismodul B um eine horizontale Achse von einer Befüllposition in eine Betriebsposition zu verschwenken, ist die Aufnahme 12 des Basismoduls B mit zwei parallelen und am Außenumfang vorgesehenen Führungsschienen 34 ausgestattet, die mit sich anschließenden Führungsschienen 36 des Steuermoduls S zusammenwirken. Die Führungsschienen 34 und 36 bilden eine kontinuierliche Führungsstruktur, mit deren Hilfe die Einheit aus Aufnahme 12, Steuermodul S und Patientenmodul P relativ zu dem Basismodul B verschwenkt werden kann.

Wie Fig. 2 zeigt, sind die Führungsschienen 34 der Schwenkaufnahme 12 mit einer Aussparung 38 versehen, mit deren Hilfe die Schwenkaufnahme 12 über zwei an dem Trägerelement 20 vorgesehene und nicht dargestellte Rollen geführt werden kann, so dass die Schwenkaufnahme 12 auf dem Trägerelement 20 des Basismoduls B verschwenkt werden kann. Die in Fig. 2 erkennbare Verzahnung dient zum Eingriff eines Dämpfungsmechanismus, der eine gleichmäßige und gedämpfte Schwenkbewegung sicherstellt.

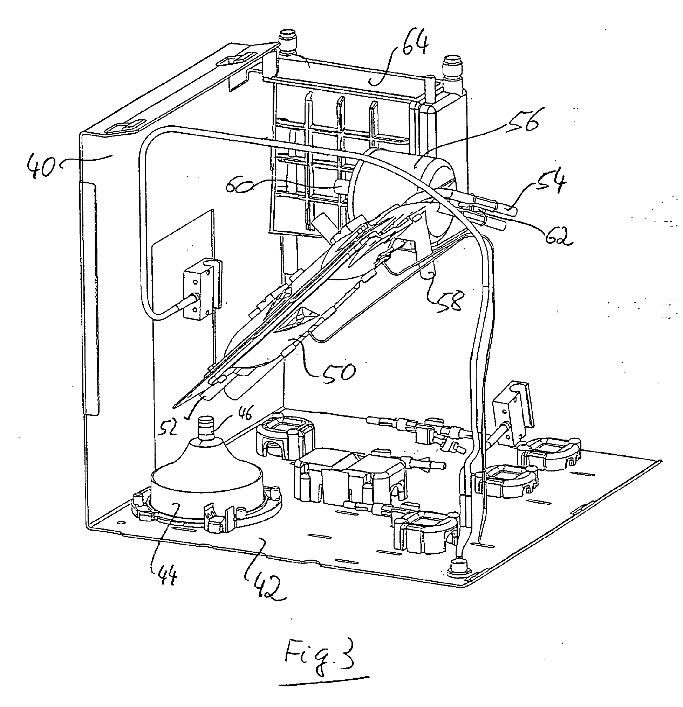

Zum Zusammenbau der Schwenkaufnahme 12 mit dem Trägerelement 20 wird die Schwenkaufnahme 12 zunächst in eine im Wesentlichen vertikale Lage gebracht und die Aussparungen 38 werden über die an dem Trägerelement 20 vorgesehenen Rollen (nicht dargestellt) geführt, woraufhin die Schwenkaufnahme 12 anschließend in die in Fig. 1 dargestellte Position verschwenkt werden kann. Nach dem Aufklappen des Bedienteils 24 kann die vorher zusammengesetzte Einheit aus Steuermodul S und Patientenmodul P auf der Schwenkaufnahme 12 verrastet werden. Um das Patientenmodul P aus der nun vorhandenen Betriebsposition in eine Befüllposition zu verschwenken, kann die nunmehr gebildete Einheit aus Steuermodul S, Patientenmodul P und Schwenkaufnahme 12 durch Herunterschwenken des Griffes 32 so um 90° verschwenkt werden, dass sich das Steuermodul S in der Position befindet, in der sich zuvor die Schwenkaufnahme 12 befunden hat. In dieser Befüllposition befinden sich die Blut führenden Komponenten des Patientenmoduls P bezogen auf die Horizontale in der in Fig. 3 dargestellten Lage und Orientierung.

Fig. 3 zeigt einige Blut führende Komponenten des Patientenmoduls, wobei das Patientenmodul P ausgehend von Fig. 1 entgegen dem Uhrzeigersinn um 90° gedreht worden ist. Die in Fig. 3 dargestellte Ansicht entspricht dabei einem Blick von der verglichen zu Fig. 1 anderen Seite auf das Patientenmodul P. Die in Fig. 3 senkrecht stehende Wand 40 des Patientenmoduls P liegt somit parallel neben der Schwenkaufnahme 12, wohingegen die horizontal orientierte Wand 42 an das Steuermodul S anschließt. Ferner sind in Fig. 3 für eine bessere Übersichtlichkeit eine Vielzahl von Schlauchverbindungen nicht dargestellt.

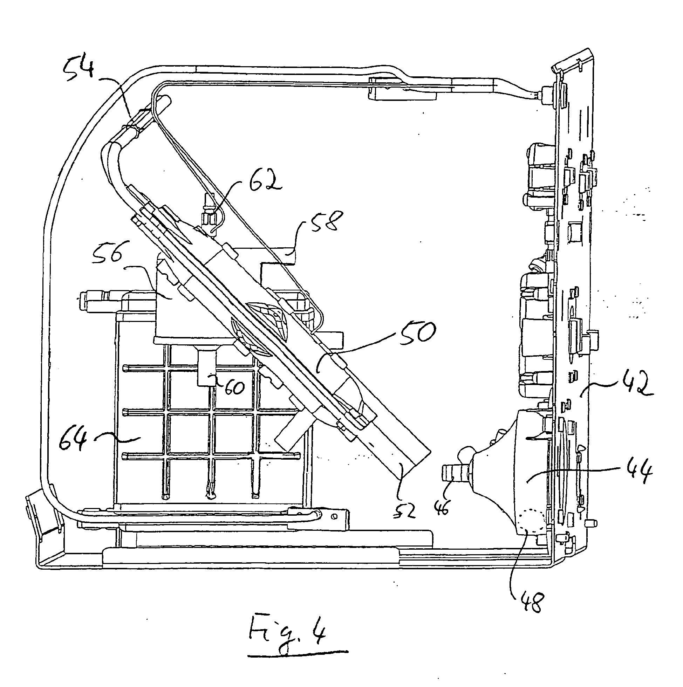

In Fig. 3 bezeichnet das Bezugszeichen 44 einen Zentrifugalpumpenkopf mit einem mittigen Saugeinlass 46 und einem radialen Auslass 48, der in Fig. 4 gestrichelt dargestellt ist.

Ferner ist in dem Patientenmodul P in einer Lage von 45° ein annähernd quaderförmiges Blutreservoir 50 eingebaut, dessen Auslass 52 über eine nicht dargestellte Schlauchleitung mit dem Einlass 46 des Zentrifugalpumpenkopfes 44 verbunden ist. An der Oberseite des Blutreservoirs 50 befinden sich Entlüfungsleitungen 54. Der von einem venösen Anschluss kommende Einlass in das Blutreservoir 50, der etwa in dessen Mitte angeordnet ist, ist in den Figuren 3 und 4 nicht erkennbar.

Ferner ist in den Figuren 3 und 4 erkennbar, dass in dem Patientenmodul P ein arterieller Filter 56 vorgesehen ist, der eine zylindrische Form besitzt, wobei ein tangentialer Einlass 58 und ein mittiger axialer Auslass 60 vorgesehen sind. An der dem Auslass 60 gegenüberliegenden Stirnfläche des Filters ist mittig ein Entlüftungsanschluss 62 vorgesehen.

Weitere dargestellte Bauteile des Patientenmoduls P sind ein Oxygenator 64 und diverse Anschlusselemente, die an der Wand 42 vorgesehen sind, die benachbart zu dem Steuermodul S liegt, und die zum Zusammenwirken mit Klemmen, Sensoren oder Steckverbindungen dienen, da sämtliche Blut führenden Komponenten in dem Patientenmodul P vorgesehen sind, wohingegen Steuerkomponenten wie Pumpenantrieb, Ventile und andere elektrische Steuerelemente in dem Steuermodul S angeordnet sind.

Fig. 4 zeigt die Darstellung von Fig. 3 in der Betriebsposition, die der Darstellung von Fig. 1 entspricht, in der das Steuermodul S und die daran anliegende Wand 42 des Patientenmoduls P vertikal orientiert sind.

Wie ein Vergleich der Figuren 3 und 4 zeigt, liegen zwischen der Befüllposition (Fig. 3) und der Betriebsposition (Fig. 4) 90°, wobei das in dem Patientenmodul P vorgesehene Blutreservoir 50 in beiden Positionen mit einer Neigung von 45° zur Horizontalen angeordnet ist, da es in dem Patientenmodul unter 45° montiert ist. Der Zentrifugalpumpenkopf 44 ist so angeordnet, dass der zentrale Einlass 46 in der Befüllposition (Fig. 3) vertikal nach oben und in der Betriebsposition (Fig. 4) horizontal zur Seite orientiert ist. Der in Fig. 3 nicht erkennbare Auslass 48 des Pumpenkopfes 44 ist in der in Fig. 4 dargestellten Betriebsposition an der untersten Stelle des Zentrifugalpumpenkopfes 44 angeordnet, so dass der Auslass 48 unterhalb des Einlasses 46 liegt.

Auch ist der arterielle Filter 56 so innerhalb des Patientenmoduls angeordnet, dass der Entlüftungsauslass 62 in der Befüllposition horizontal und in der Betriebsposition (Fig. 4) vertikal nach oben orientiert ist. Der Einlass 58 ist in der Befüllposition vertikal nach unten und in der Betriebsposition horizontal orientiert, wohingegen der Auslass 60 in der Befüllposition horizontal und in der Betriebsposition vertikal nach unten orientiert ist.

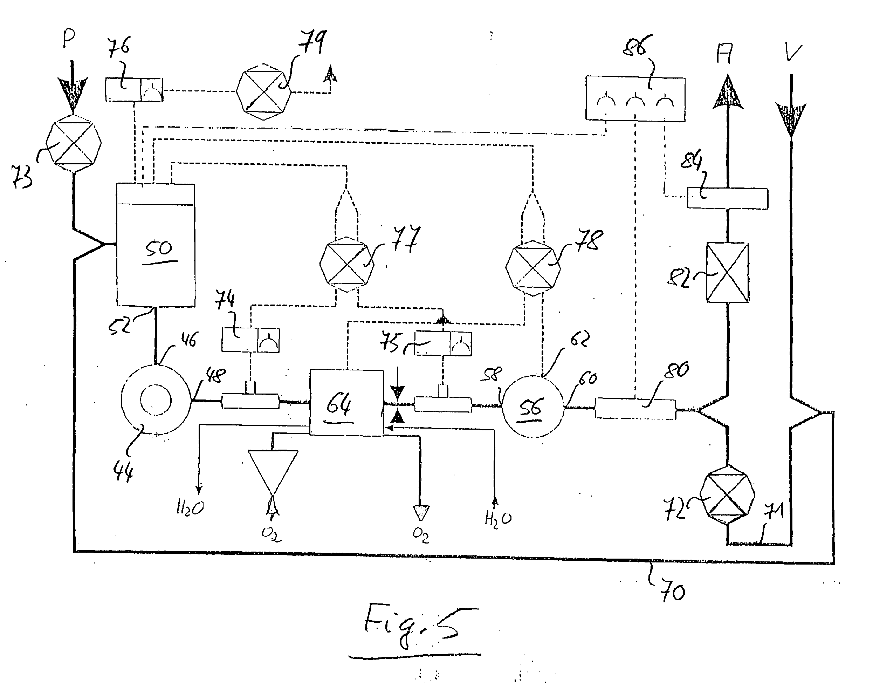

Fig. 5 zeigt die verschiedenen Komponenten der erfindungsgemäßen Herz-Lungen-Maschine, bei der das von einem venösen Anschluss V kommende Patientenblut über eine Leitung 70 in das Blutreservoir 50 geleitet wird und von dort über den Auslass 52 in den Einlass 46 der Zentrifugalpumpe 44 gelangt. Von dort wird es über den Auslass 48 in den Oxygenator 64 gepumpt und gelangt von dort über den arteriellen Filter 56 zum arteriellen Anschluss A und von dort zurück in den Körper des Patienten. Mit dem Bezugszeichen 71 ist ein interner Bypass bezeichnet, der über ein Ventil 72 schaltbar ist. Das Bezugszeichen 73 bezeichnet ein Ventil für die Zuleitung PR, mit der Priming-Flüssigkeit in den Kreislauf geführt werden kann. Die Bezugszeichen 74, 75 und 76 bezeichnen jeweils Drucksensoren. Mit den Bezugszeichen 77, 78 und 79 sind Entlüftungsventile bezeichnet, wobei die Ventile 77 und 78 die Entlüftungswege in den oberen, nicht mit Blut gefüllten Bereich des Blutreservoirs 50 schalten und das Entlüftungsventil 79 die Entlüftung aus dem Blutreservoir steuert. Das Bezugszeichen 80 bezeichnet einen Blasensensor, der ein im arteriellen Ausgang A vorgesehenes Schnellschlussventil 82 ansteuert, falls Luftblasen detektiert werden. Das Bezugszeichen 84 bezeichnet einen Flusssensor und das Bezugszeichen 86 eine elektrische Schnittstelle.

Wie die Figur ferner zeigt, ist der Oxygenator 64 mit Zu- und Ableitungen für Wasser und Sauerstoff versehen, um eine Anreicherung des Blutes mit Sauerstoff und eine Temperierung des Blutes zu bewirken.

Zur Inbetriebnahme der oben beschriebenen Herz-Lungen-Maschine wird ausgehend von der Darstellung von Fig. 1 zunächst der Klappbügel 14 um 180° nach unten geschwenkt und das Bedienteil 24 wird nach links geklappt. Anschließend kann die gesamte Einheit bestehend aus Steuermodul S, Patientenmodul P und Schwenkaufnahme 12 entgegen dem Uhrzeigersinn verschwenkt werden, so dass die Befüllposition erreicht ist.

In der Befüllposition wird über den Anschluss PR Priming-Flüssigkeit zugeführt, die zunächst (vgl. Fig. 5) das Blutreservoir und von dort den Zentrifugalpumpenkopf 44 füllt. Durch die beim Befüllen oberhalb der Maschine angeordnete Priming-Flüssigkeit wird hierbei die in der Verschlauchung befindliche Luft weitgehend aus dem System entfernt, wobei jedoch im oberen Bereich des arteriellen Filters 56 und in horizontalen Leitungsteilen Lufteinschlüsse verbleiben.

Wenn das Blutreservoir 50 nahezu gefüllt ist, wird der Zentrifugalpumpenkopf 44 vergleichsweise langsam in Rotation versetzt, wodurch die Priming-Flüssigkeit durch das System gepumpt wird und weitere Luftreste aus dem System entfernt werden. Nach einer Zeitdauer von etwa 20 Sekunden sind auch weitere Komponenten wie beispielsweise der Oxygenator 64 mit Priming-Flüssigkeit gefüllt, so dass die Pumpe angehalten und die Einheit aus Steuermodul S, Patientenmodul P und Schwenkaufnahme 12 in die Betriebsposition zurückgeschwenkt werden kann. Nach diesem Zurückschwenken um 90° kann auch noch diejenige Luft entweichen, die in dem arteriellen Filter 56 und in horizontalen Leitungsteilen verblieben war. Somit kann innerhalb eines Zeitraums in der Größenordnung von etwa 2 Minuten ein komplettes Befüllen und Entlüften des Patientenmoduls erreicht werden.

### Bezugszeichenliste

- 10: Gerätefuß
- 12: Schwenkaufnahme
- 14: Klappbügel
- 16, 17: Halteklips
- 20: Trägerelement
- 22: Steckbuchse
- 24: Bedienteil
- 26: Touchscreen
- 28, 30: Rastverbindungen
- 32: Griff
- 34, 36: Führungsschiene
- 38: Aussparung
- 40, 42: Wand
- 44: Zentrifugalpumpenkopf
- 46: Einlass
- 48: Auslass
- 50: Blutreservoir
- 52: Auslass
- 54: Entlüftungsleitung
- 56: arterieller Filter
- 58: Einlass
- 60: Auslass
- 62: Entlüftungsöffnung
- 64: Oxygenator
- 70: Leitung
- 71: interner Bypass
- 72, 73: Ventil
- 74, 75, 76: Drucksensor
- 77, 78, 79: Ventil
- 80: Blasensensor
- 82: Schnellschlussklemme
- 84: Flusssensor
- 86: elektrische Schnittstelle

- A: arterieller Anschluss
- B: Basismodul
- P: Patientenmodul
- PR: Priminganschluss
- S: Steuermodul
- V: venöser Anschluss

## Patentansprüche

1. Vorrichtung zur Bereitstellung eines extrakorporalen Blutkreislaufs, insbesondere Herz-Lungenmaschine, umfassend zumindest ein Basismodul (B) mit einer Steuereinrichtung (24) und ein mit dem Basismodul lösbar verbundenes Patientenmodul (P), das Blut führende Komponenten des extrakorporalen Blutkreislaufs aufweist,
**dadurch gekennzeichnet, dass**
an dem Basismodul (B) und/oder an dem Patientenmodul (P) Schwenkmittel (12, 34, 36) vorgesehen sind, um das Patientenmodul (P) relativ zu dem Basismodul (B) um eine horizontale Achse von einer Befüllposition in eine Betriebsposition zu verschwenken.

2. Vorrichtung nach Anspruch 1,
**dadurch gekennzeichnet, dass**
zwischen der Befüllposition und der Betriebsposition etwa 90° liegen.

3. Vorrichtung nach Anspruch 1 oder 2,
**dadurch gekennzeichnet, dass**
in dem Patientenmodul (P) ein Blutreservoir (50) vorgesehen ist, das in der Befüllposition und in der Betriebsposition mit einer Neigung von etwa 45° zur Horizontalen angeordnet ist.

4. Vorrichtung nach zumindest einem der vorstehenden Ansprüche,
**dadurch gekennzeichnet, dass**
in dem Patientenmodul (P) ein Zentrifugalpumpenkopf (44) mit zentralem Einlass (46) und tangentialem Auslass (48) so angeordnet ist, dass der Einlass (46) in der Befüllposition vertikal nach oben und in der Betriebsposition horizontal orientiert ist.

5. Vorrichtung nach zumindest einem der vorstehenden Ansprüche,
**dadurch gekennzeichnet, dass**
in dem Patientenmodul ein Zentrifugalpumpenkopf (44) mit tangentialem Auslass (48) so angeordnet ist, dass der Auslass (48) in der Betriebsposition an der untersten Stelle des Zentrifugalpumpenkopfes (44) angeordnet ist.

6. Vorrichtung nach zumindest einem der vorstehenden Ansprüche,
**dadurch gekennzeichnet, dass**
in dem Patientenmodul (P) ein arterieller Filter (56) mit einem Entlüftungsauslass (62) so angeordnet ist, dass der Entlüftungsauslass (62) in der Befüllposition horizontal und in der Betriebsposition vertikal nach oben orientiert ist.

7. Vorrichtung nach zumindest einem der vorstehenden Ansprüche,
**dadurch gekennzeichnet, dass**
die Schwenkmittel eine verschwenkbar an dem Basismodul (B) gelagerte Aufnahme (12) für das Patientenmodul (P) umfassen.

8. Vorrichtung nach Anspruch 7,
**dadurch gekennzeichnet, dass**
die Schwenkmittel eine Führung (34, 36) umfassen, die an der Aufnahme (12) und an dem Patientenmodul (P) und/oder an einem mit dem Patientenmodul verbundenen weiteren Modul (S) vorgesehen ist.

9. Vorrichtung nach zumindest einem der vorstehenden Ansprüche,
**dadurch gekennzeichnet, dass**
sich das Patientenmodul (P) nach Aufsetzen auf das Basismodul (B) in der Betriebsposition befindet.

10. Vorrichtung nach zumindest einem der vorstehenden Ansprüche,
**dadurch gekennzeichnet, dass**
das Basismodul (B) einen Gerätefuß (10) aufweist, der mit einem Klappbügel (14) versehen ist, um die Vorrichtung an dem Rahmen eines Patientenbettes einzuhängen.

11. Verfahren zur Inbetriebnahme einer Vorrichtung zur Bereitstellung eines extrakorporalen Blutkreislaufs, insbesondere einer Herz-Lungenmaschine, umfassend zumindest ein Basismodul mit einer Steuereinrichtung und ein mit dem Basismodul lösbar verbundenes Patientenmodul, das Blut führende Komponenten des extrakorporalen Blutkreislaufs aufweist, wobei an dem Basismodul und/oder an dem Patientenmodul Schwenkmittel vorgesehen sind, um das Patientenmodul relativ zu dem Basismodul um eine horizontale Achse von einer Befüllposition in eine Betriebsposition zu verschwenken, bei welchem Verfahren das Patientenmodul zunächst in die Befüllposition gebracht wird, in dieser die Blut führenden Komponenten mit einer Primingflüssigkeit befüllt werden, und wobei anschließend das Patientenmodul relativ zu dem Basismodul, insbesondere um 90°, in die Betriebsposition verschwenkt wird.

12. Verfahren nach Anspruch 11,
**dadurch gekennzeichnet, dass**
vor dem Verschwenken ein in dem Patientenmodul vorgesehener Pumpenkopf angetrieben wird, um die Blut führenden Komponenten zu entlüften.

## Claims

1. An apparatus for the provision of an extracorporeal blood circuit, in particular a heart-lung machine, comprising at least one base module (B) having a control device (24) and a patient module (P) releasably connected to the base module and having blood-conducting components of the extracorporeal blood circuit,
**characterized in that**
pivot means (12, 34, 36) are provided at the base module (B) and/or at the patient module (P) to pivot the patient module (P) relative to the base module (B) about a horizontal axis from a filling position into an operating position.

2. An apparatus in accordance with claim 1, **characterized in that** there is approximately 90° between the filling position and the operating position.

3. An apparatus in accordance with claim 1 or claim 2, **characterized in that** a blood reservoir (50) is provided in the patient module (P) which is arranged at an inclination of approximately 45° to the horizontal in the filling position and in the operating position.

4. An apparatus in accordance with at least one of the preceding claims, **characterized in that** a centrifugal pump head (44) having a central inlet (46) and a tangential outlet (48) is arranged in the patient module (P) such that the inlet (46) is oriented vertically upwardly in the filling position and horizontally in the operating position.

5. An apparatus in accordance with at least one of the preceding claims, **characterized in that** a centrifugal pump head (44) having a tangential outlet (48) is arranged in the patient module such that the outlet (48) is arranged at the bottommost position of the centrifugal pump head (44) in the operating position.

6. An apparatus in accordance with at least one of the preceding claims, **characterized in that** an arterial filter (56) having a venting outlet (62) is arranged in the patient module (P) such that the venting outlet (62) is oriented horizontally in the filling position and vertically upwardly in the operating position.

7. An apparatus in accordance with at least one of the preceding claims, **characterized in that** the pivot means include a mount (12) for the patient module (P) pivotally supported at the base module (B).

8. An apparatus in accordance with claim 7, **characterized in that** the pivot means include a guide (34, 36) which is provided at the mount (12) and at the patient module (P) and/or at a further module (S) connected to the patient module.

9. An apparatus in accordance with at least one of the preceding claims, **characterized in that** the patient module (P) is in the operating position after mounting onto the base module (B).

10. An apparatus in accordance with at least one of the preceding claims, **characterized in that** the base module (B) has a device stand (10) which is provided with a pivotal hook (14) to hang the apparatus to the frame of a patient's bed.

11. A method for the putting into operation of an apparatus for the provision of an extracorporeal blood circuit, in particular of a heart-lung machine, comprising at least one base module having a control device and a patient module releasably connected to the base module and comprising blood-conducting components of the extracorporeal blood circuit, with pivot means being provided at the base module and/or at the patient module to pivot the patient module relative to the base module about a horizontal axis from a filling position into an operating position, in which method the patient module is first brought into the filling position, the blood-conducting components are filled with a priming liquid in said filling position, and with the patient module subsequently being pivoted into the operating position relative to the base module, in particular by 90°.

12. A method in accordance with claim 11, **characterized in that** a pump head provided in the patient module is driven prior to the pivoting to vent the blood-conducting components.

## Revendications

1. Dispositif pour établir un circuit sanguin extracorporel, en particulier coeur-poumon artificiel, comprenant au moins un module de base (B) avec une unité de commande (24) et un module de patient (P) relié de façon détachable au module de base, qui comprend les composants du circuit sanguin extracorporel qui mènent le sang,
**caractérisé en ce que**
sur le module de base (B) et/ou sur le module de patient (P) sont prévus des moyens de pivotement (12, 34, 36) pour faire pivoter le module de patient (P) par rapport au module de base (B) autour d'un axe horizontal depuis une position de remplissage jusque dans une position de service.

2. Dispositif selon la revendication 1,
**caractérisé en ce que** 90° environ séparent la position de remplissage de la position de service.

3. Dispositif selon la revendication 1 ou 2,
**caractérisé en ce qu'**un réservoir à sang (50) est prévu dans le module de patient (P), lequel est agencé dans la position de remplissage et dans la position de service avec une inclinaison d'environ 45° par rapport à l'horizontale.

4. Dispositif selon l'une au moins des revendications précédentes,
**caractérisé en ce qu'**une tête de pompe centrifuge (44) avec entrée centrale (46) et sortie tangentielle (48) est agencée dans le module de patient (P) de telle façon que l'entrée (46) est orientée verticalement vers le haut dans la position de remplissage et horizontalement dans la position de service.

5. Dispositif selon l'une au moins des revendications précédentes,
**caractérisé en ce qu'**une tête de pompe centrifuge (44) avec sortie tangentielle (48) est agencée dans le module de patient de telle façon que la sortie (48) est agencée dans la position de service à l'emplacement le plus bas de la tête de pompe centrifuge (44).

6. Dispositif selon l'une au moins des revendications précédentes,
**caractérisé en ce qu'**un filtre artériel (56) avec sortie de purge d'air (62) est agencé dans le module de patient (P) de telle façon que la sortie de purge d'air (62) est orientée horizontalement dans la position de remplissage et verticalement vers le haut dans la position de service.

7. Dispositif selon l'une au moins des revendications précédentes,
**caractérisé en ce que** les moyens de pivotement comprennent un logement (12), pour le module de patient (P), monté en pivotement sur le module de base (B).

8. Dispositif selon la revendication 7,
**caractérisé en ce que** les moyens de pivotement comprennent un guidage (34, 36) qui est prévu sur le logement (12) et sur le module de patient (P) et/ou sur un autre module (S) relié au module de patient.

9. Dispositif selon l'une au moins des revendications précédentes,
**caractérisé en ce que** le module de patient (P), après sa pose sur le module de base (B), se trouve dans la position de service.

10. Dispositif selon l'une au moins des revendications précédentes,
**caractérisé en ce que** le module de base (B) comprend un pied d'appareil (10), qui est doté d'un étrier rabattable (14) pour accrocher le dispositif sur le cadre d'un lit de patient.

11. Procédé pour la mise en service d'un dispositif pour établir un circuit sanguin extracorporel, en particulier coeur-poumon artificiel, comprenant au moins un module de base avec une unité de commande et un module de patient, relié de façon détachable au module de base, qui comprend les composants du circuit sanguin extracorporel qui mènent le sang, dans lequel des moyens de pivotement sont prévus sur le module de base et/ou sur le module de patient pour faire pivoter le module de patient par rapport au module de base autour d'un axe horizontal depuis une position de remplissage jusque dans une position de service, procédé dans lequel le module de patient est tout d'abord amené dans la position de remplissage, dans celle-ci les composants qui mènent le sang sont remplis d'un liquide d'amorçage, et dans lequel le module de patient est ensuite pivoté par rapport au module de base, en particulier de 90°, jusque dans la position de service.

12. Procédé selon la revendication 11,
**caractérisé en ce qu'**avant le pivotement, une tête de pompe prévue dans le module de patient est entraînée pour purger l'air hors des composants qui mènent le sang.
